# EUROPEAN PATENT SPECIFICATION

(11) **EP 1 368 298 B1**
(45) Date of publication and mention of the grant of the patent: **04.04.2007**
(21) Application number: 00985257.5
(22) Date of filing: 23.12.2000
(51) Int. Cl.: C07C 67/343, C07C 69/88

(54) **PROCESS FOR THE PREPARATION OF 5- AND/OR 6 - SUBSTITUTED - 2 - HYDROXY-BENZOIC ACID ESTERS**
VERFAHREN ZUR HERSTELLUNG VON 5- UND/ODER 6-SUBSTITUIERTEN 2-HYDROXYBENZOESÄUREESTERN
PROCEDE DE PREPARATION D'ESTERS D'ACIDE 2 - HYDROXY-BENZOIQUE SUBSTITUES EN 5 ET/OU 6

(43) Date of publication of application: 10.12.2003
(73) Proprietor: BASF Aktiengesellschaft, 67056 Ludwigshafen (DE)
(72) Inventor: PFRENGLE, Andreas, 55411 Bingen (DE); SCHEFFER, Robert, J., H., 55218 Ingelheim (DE); SCHEIBLICH, Stefan, Dr., 82377 Penzberg (DE); WEVERS, Jan, Hendrik, 55252 Mainz (DE); VOGELBACHER, Uwe, Josef, 67071 Ludwigshafen (DE); DOEHNER, Robert, F., East Windsor, NJ 08520 (US)
(74) Representative: Isenbruck, Günter
(86) International application number: PCT/EP2000/013259
(87) International publication number: WO 2002/051790

(56) References cited:
- WO-A-01/05739
- FRANK M HAUSER: "2-Hydroxy-6-methylbenzoic Acid Derivatives" SYNTHESIS,GEORG THIEME VERLAG. STUTTGART,DE, no. 10, October 1980 (1980-10), pages 814-815, XP002149544 ISSN: 0039-7881 cited in the application

## Description

### Background of the invention

Derivatives of 2-hydroxybenzoic acid esters are useful starting materials for natural product synthesis or for the manufacture of fungicidal benzophenones such as those described in U.S. 5,773,663. Methods to prepare said 2-hydroxybenzoic acid esters are known, i.e. F.M. Hauser et al., Synthesis, 1980, 814 or Y. Hamada, et al., Tetrahedron, Vol. 47 (1991), 8635. However, these known methods require several steps and utilize corrosive or toxic reagents and are not amenable to large scale preparation or commercial manufacturing conditions.

The two-step syntheses cited in Synthesis and Tetrahedron hereinabove require the isolation of intermediates resulting in an undue solvent waste load on the environment. Further these syntheses require gaseous HCl and a separate oxidation procedure employing oxidizing reagents such as Br₂ or CuCl₂.

Therefore, it is an object of this invention to provide an effective and efficient single-step process to prepare 5- and/or 6-substituted-2-hydroxybenzoic acid esters which is amenable to large scale preparations and commercial manufacturing procedures.

It is another object of this invention to provide an effective means of obtaining a substituted-2-hydroxybenzoic acid ester in good yield under relatively mild reaction conditions from readily available starting materials and reagents.

These and other objects and features of the invention will become more apparent from the detailed description set forth hereinbelow.

### Summary of the invention

The present invention provides a single-step process for the preparation of a compound of formula I 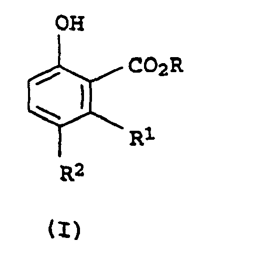 wherein R is C₁-C₆ alkyl; and
R¹ and R² are each independently H or C₁-C₄ alkyl which process comprises reacting a compound of formula II 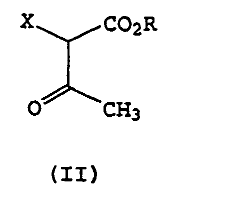 wherein R is C₁-C₆ alkyl and X is halogen or OCOCH₃ with a compound of formula III 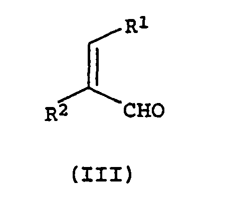 wherein R¹ and R² are each independently H or C₁-C₄ alkyl, and formula III compound is understood to be cis, trans, or a mixture thereof, in the presence of a C₁-C₄ carboxylic acid salt and a solvent, wherein the molar ratio of starting marterials: compound III to compound II is higher or equal to 1.8.

### Detailed description of the invention

Substituted-2-hydroxybenzoic acid esters of formula I are useful as key starting materials in natural product synthesis and in the manufacture of important benzophenone fungicidal agents. Therefore, the efficient preparation of such fungicidally active compounds in an environmentally sound manner is highly desirable.

The present single-step process makes salicylic acid of the formula I available from β-ketoesters of the formula II and aldehydes of the formula III. Its practicability is ensured by using the aldehyde III in a molar ratio of not less than 1.8 relative to the compound II. This ensures that the reaction mixture remains efficiently stirrable even at low solvent quantities and consistently good yields are obtainable even on an industrial scale.

In a preferred embodiment of the process, the aldehyde III is initially charged and subsequently the salt and the solvent are added in succession or concurrently in the course of generally 0 to 3 hours.

It can further be of advantage to raise the reaction temperature in the course of the reaction from initially 60-120°C to finally 130-140°C.

Preferred compounds prepared by the process of the invention are those compounds of formula I wherein R¹ is C₁-C₄ alkyl and R² is hydrogen. More preferred compounds are those compounds of formula I wherein R¹ is methyl and R² is hydrogen.

Preferred compounds of formula II employed in the process of the invention are those compounds wherein X is halogen. More preferred compounds are those compounds of formula II wherein X is C1.

Compounds of formula III may be represented in the cis or trans configuration or as a mixture thereof. In the specification and claims, compounds designated as formula III include the cis isomer, the trans isomer or a mixture thereof.

The term halogen as used in the specification and claims designates Cl, Br, F or I.

In accordance with the process of the invention, a β-ketoester of formula II is reacted with an α,β-unsaturated aldehyde of formula III in the presence of a C₁-C₄ carboxylic acid salt and a solvent to form the desired product of formula I. The reaction is shown in flow diagram I wherein M is an alkali metal or an alkaline-earth metal.

### Flow diagram I

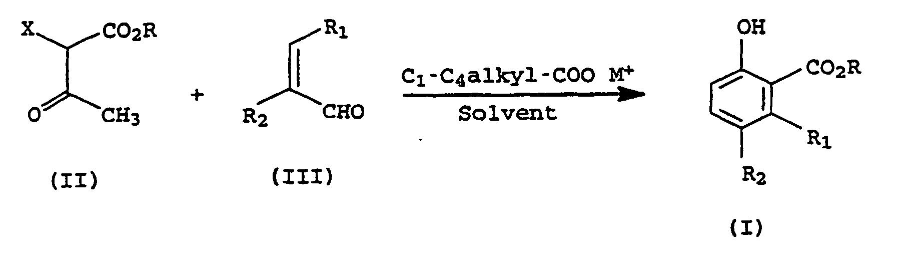

Suitable solvents for use in the inventive process include polar solvents, preferably protic solvents such as C₁-C₄ carboxylic acids or C₁-C₆ alkanols. Preferred solvents are C₂-C₄ carboxylic acids or mixtures of a C₂-C₄ carboxylic acid and a C₁-C₆ alkanol, more preferably acetic acid or a mixture thereof with methanol or ethanol. In general, more than 2.5 molar equivalents of solvent; preferably about 2.5 to 5 and more preferably about 2.5 to 3.5 molar equivalents of solvent were used.

Acid salts suitable for use in the process of the invention are C₁-C₄ carboxylic acid alkali metal or alkaline-earth salts, more preferably acetic acid alkali metal salts such as sodium acetate or potassium acetate. Preferably about 1.3 to 3.0 molar equivalents, more preferably about 1.4 to 1.6 molar equivalents of the carboxylic acid salt were added to the reaction mixture.

In the present process, the aldehyde III is used in a molar ratio of not less than 1.8 relative to the compound II. Preferably about 1.9 to 2.5 and more preferably about 2.0 to 2.2 molar equivalents of aldehyde III were reacted.

In a preferred embodiment of the process, the aldehyde III is initially charged and subsequently the salt and the solvent are added in succession or concurrently in the course of generally 0 to 3 hours. It can be of advantage here first to add a portion or the total amount of the salt and only then to start with the addition of the solvent. Compound II is generally added last in the course of 0 to 3 hours.

In the process of the invention, reaction rate is directly related to reaction temperature, that is, the reaction rate increases with increased temperature. However, excessively high reaction temperatures may lead to decomposition and the formation of undesired by-products, thereby reducing product yield and purity. Suitable reaction temperatures in the process of the invention may range from room temperature to the reflux temperature of the solvent preferably about 60°C to 150°C, more preferably about 110°C to 140°C.

It can further be of advantage to raise the reaction temperature in the course of the reaction from initially 60-120°C to finally 130-140°C. The reaction temperature can be raised for example by removing low boilers, if necessary by applying reduced pressure. It is similarly possible to carry out the reaction in a closed system. In this case, a higher reaction temperature will automatically result in a higher pressure. The pressure employed is preferably in the range from 0.1 to 6 atm.

The individual reactants are generally added at room temperature to the reflux temperature of the solvent.

The reaction time is generally in the range from 3 to 8 hours.

The formula I hydroxybenzoic acid ester product may be isolated using conventional isolation techniques such as precipitation, decantation, filtration, extraction, chromatographic separation or the like, preferably filtration or extraction.

Compounds of formula I are useful as intermediates in the synthesis of natural products and in the manufacture of benzophenone fungicidal agents as described in U.S. 5,773,663.

For a more clear understanding of the invention, the following example is set forth below. This example is merely illustrative and is not be understood as limiting the scope or underlying principles of the invention in any way.

The term NMR designates nuclear magnetic resonance spectroscopy. Unless otherwise mentioned, all parts are parts by weight.

### Example 1

### Preparation of Ethyl 2-hydroxy-6-methylbenzoate

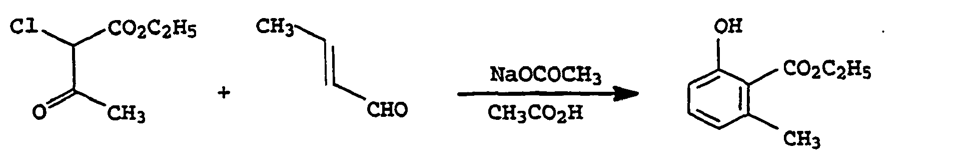

A 4 l jacketed vessel fitted with a destillation apparatus was initially charged with 770.9 g [11 mol] of crotonaldehyde. With stirring, 615.2 g [7.5 mol] of anhydrous sodium acetate were added and the batch was heated to 60°C. 900 g of acetic acid were then metered in over an hour, during which the temperature rose to 83°C. The suspension was then heated under reflux and admixed with 857.2 g [5 mol] of ethyl 2-chloroacetate (purity: 96%) in the course of an hour.

The batch was subsequently stirred for five hours, during which initially about 700 ml of low boilers were destilled off until an internal temperature of 130°C was attained. The pressure was then reduced to 200 mbar and the distallation continued until the internal temperature again reached 135°C.

The residue was cooled down to 85°C and admixed with 1250 g of water (preheated to 80°C). After 10 min of stirring at 80°C the aqueous phase was separated off. This afforded 893 g of organic phase as a dark brown oil having a product content of 63.2% (which corresponds to a yield of 62.6% of theory).

## Claims

1. A process for the preparation of a compound of formula I 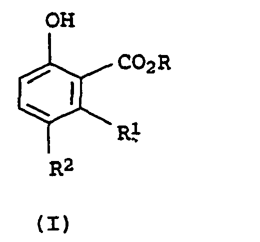 wherein R is C₁-C₆ alkyl; and
R¹ and R² are each independently H or C₁-C₄ alkyl which process comprises reacting a compound of formula II 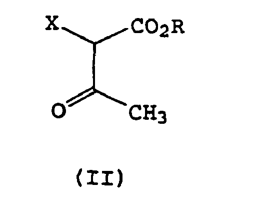 wherein R is C₁-C₆ alkyl and X is halogen or OCOCH₃ with a compound of formula III 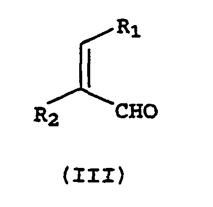 wherein R¹ and R² are each independently H or C₁-C₄ alkyl, in the presence of a C₁-C₄ carboxylic acid salt and a solvent, wherein the molar ratio of compound III : compound II is higher or equal to 1.8.

2. The process according to claim 1 wherein the aldehyde III is initially charged, the salt and the solvent are subsequently added in succession or concurrently and finally compound II is added.

3. The process according to claim 1 wherein a reaction temperature of initially 60°C to the reflux temperature of the solvent used is set and the temperature is raised to 130-140°C in the course of the reaction.

4. The process according to claim 1 wherein the solvent is a C₁-C₄ carboxylic acid or a mixture thereof with a C₁-C₆ alkanol.

5. The process according to claim 2 wherein the solvent is acetic acid and the C₁-C₄ carboxylic acid salt is sodium acetate.

6. The process according to claim 1 having a formula II compound wherein X is Cl.

7. The process according to claim 1 having a formula III compound wherein R¹ is methyl and R² is H.

## Patentansprüche

1. Verfahren zur Herstellung einer Verbindung der Formel I, 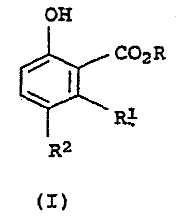 in der R C₁-C₆-Alkyl bedeutet und
R¹ und R² jeweils unabhängig H oder C₁-C₄-Alkyl bedeuten, bei dem man eine Verbindung der Formel II, 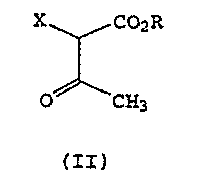 in der R C₁-C₆-Alkyl bedeutet und X Halogen oder OCOCH₃ bedeutet, mit einer Verbindung der Formel III, 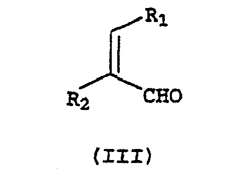 in der R¹ und R² jeweils unabhängig H oder C₁-C₄-Alkyl bedeuten, in Gegenwart eines C₁-C₄-Carbonsäuresalzes und eines Lösungsmittels umsetzt, wobei das molare Verhältnis von Verbindung III zu Verbindung II 1,8 oder mehr beträgt.

2. Verfahren nach Anspruch 1, wobei der Aldehyd III vorgelegt wird, anschließend das Salz und das Lösungsmittel entweder nacheinander oder gleichzeitig zugegeben werden und schließlich die Verbindung II zugegeben wird.

3. Verfahren nach Anspruch 1, wobei eine Reaktionstemperatur von ursprünglich 60°C bis zur Rückflußtemperatur des verwendeten Lösungsmittels eingestellt wird und die Temperatur im Lauf der Reaktion auf 130-140°C angehoben wird.

4. Verfahren nach Anspruch 1, wobei es sich bei dem Lösungsmittel um eine C₁-C₄-Carbonsäure oder eine Mischung davon mit einem C₁-C₆-Alkanol handelt.

5. Verfahren nach Anspruch 2, wobei es sich bei dem Lösungsmittel um Essigsäure und bei dem C₁-C₄-Carbonsäuresalz um Natriumacetat handelt.

6. Verfahren nach Anspruch 1 mit einer Verbindung der Formel II, bei der X C1 bedeutet.

7. Verfahren nach Anspruch 1 mit einer Verbindung der Formel III, bei der R¹ Methyl bedeutet und R² H bedeutet.

## Revendications

1. Procédé de préparation d'un composé selon la formule I 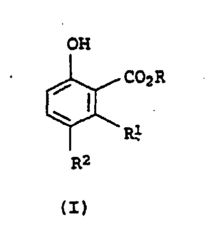 dans laquelle R est un alkyle en C₁-C₆ ; et
R¹ et R² sont, indépendamment l'un de l'autre, H ou un alkyle en C₁-C₄, lequel procédé comprend la réaction d'un composé selon la formule II 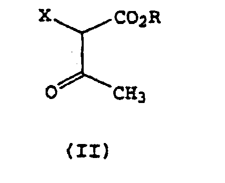 dans laquelle R est un alkyle en C₁-C₆ et X est un halogène ou OCOCH₃, avec un composé selon la formule III 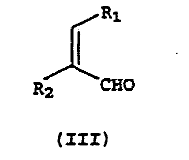 dans laquelle R¹ et R² sont, indépendamment l'un de l'autre, H ou un alkyle en C₁-C₄, en présence d'un sel d'acide carboxylique en C₁-C₄ et d'un solvant,
dans laquelle le rapport molaire composé III : composé II est supérieur ou égal à 1,8.

2. Procédé selon la revendication 1 dans lequel l'aldéhyde III est initialement chargé, le sel et le solvant sont ensuite ajoutés successivement ou simultanément et le composé II est finalement ajouté.

3. Procédé selon la revendication 1 dans lequel une température initiale de réaction de 60°C à la température de reflux du solvant utilisé est réglée et la température est portée à 130 à 140°C pendant la réaction.

4. Procédé selon la revendication 1 dans lequel le solvant est un acide carboxylique en C₁-C₄ ou un mélange de ce dernier avec un alcanol en C₁-C₆.

5. Procédé selon la revendication 2 dans lequel le solvant est un acide acétique et le sel d'acide carboxylique en C₁-C₄ est un acétate de sodium.

6. Procédé selon la revendication 1 incluant un composé selon la formule II dans laquelle X est C₁.

7. Procédé selon la revendication 1 incluant un composé selon la formule III dans laquelle R¹ est un méthyle et R² est H.
